# EUROPEAN PATENT SPECIFICATION

(11) **EP 2 801 352 B1**
(45) Date of publication and mention of the grant of the patent: **05.07.2017**
(21) Application number: 14167375.6
(22) Date of filing: 07.05.2014
(51) Int. Cl.: A61K 9/14, A61K 9/50, A61K 9/00, A61K 31/165, A61K 9/20

(54) **Orally disintegrating formulations of Lacosamid**
Im Mund zerfallende Formulierung von Lacosamid
Formulation à désintégration orale de lacosamide

(30) Priority: 08.05.2013 TR 201305490; 13.05.2013 TR 201305664
(43) Date of publication of application: 12.11.2014
(73) Proprietor: Sanovel Ilac Sanayi ve Ticaret A.S., 34460 Istanbul (TR)
(72) Inventor: Türkyilmaz, Ali, 34460 Istanbul (TR); TURP, Ali Hasan, 34460 Istanbul (TR); Kirat Uzunogullar, Nur, 34460 Istanbul (TR); Gülkok, Yildiz, 34460 Istanbul (TR); Saydam, Mehtap, 34460 Istanbul (TR); Eceoglu, Melike, 34460 Istanbul (TR)
(74) Representative: Sevinç, Erkan

(56) References cited:
- EP-A1- 2 468 261
- WO-A1-2009/146325
- WO-A1-2011/055385
- WO-A2-2010/060624
- WO-A2-2011/130615

## Description

### Field of Invention

The present invention relates to a pharmaceutical formulation comprising a therapeutically effective amount of lacosamide or a pharmaceutically acceptable salt thereof in the form of orally disintegrating tablet.

### Background of Invention

Lacosamide is an aminoacid derivative with an anticonvulsant activity useful in the adjunctive treatment of partial-onset seizures with or without secondary generalization in adults with epilepsy. It is also called as erlosamide or harkoseride. Its chemical name is (2R)-2-(Acetylamino)-3-methoxy-N-(phenylmethyl) propanamide and its chemical structure is shown in the Formula I.

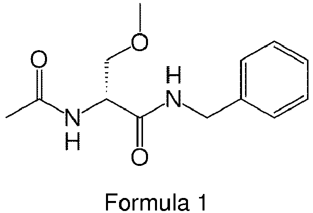

The precise mechanism of antiepileptic effects of lacosamide in humans remains to be fully elucidated. In vitro electrophysiological studies have shown that lacosamide selectively enhances slow inactivation of voltage-gated sodium channels, resulting in stabilization of hyperexcitable neuronal membranes and inhibition of repetitive neuronal firing. Inactivation of sodium channels is important to control of abnormal neuronal activity in the brain of epilepsy patient, and it can occur via fast or slow mechanism. While tranditional sodium channel blocking antiepileptics (e.g., Lamotrigine, Carbamazepine, Oxcarbazepine or Phenytoin) act primarly via fast inactivation, lacosamide acts by selectively enhancing slow inactivation of voltage-gated sodium channels to control and stabilise the neural network in the brain.

Lacosamide is sparingly soluble in water and it has low flowability, which makes the tableting process difficult at the high amount of active substance in tablets.

There is lacosamide immediate release formulation licensed in the US and Europe in the form of immediate release tablets, oral solutions and intravenous injection solutions under the brand name Vimpat® by UCB. Vimpat® IR tablet has crospovidone as disintegrant agent, microcrystalline cellulose, HPC (low substituted) and HPC as filler and binder, silicified microcrystalline cellulose as glidant, colloidal silicon dioxide as filler, magnesium stearate as lubricant and a non-functional coating.It may be taken with or without food. The initial dose should be 50 mg twice daily (100 mg per day). Vimpat® IR tablet is prepared by wet granulation. To overcome poor flowability of lacosamide, wet granulation comprising HPC (including low substituted HPC) is selected.

Lacosamide can be increased at weekly intervals by 100 mg/day given as two divided doses up to the recommended maintenance dose of 200 to 400 mg/day, based on individual patient response and tolerability. The currently commercially available tablets are available in strengths of 50, 100, 150 and 200 mg of lacosamide.

In prior art, there are several patents which disclose orally disintegrating compositions but none of them includes lacosamide or a pharmaceutically acceptable salt thereof.

Over the past decades, orally disintegrating tablets (ODTs) have gained considerable attention as a preferred alternative to conventional tablets and capsules due to better patient compliance. ODTs are solid dosage forms containing active ingredients which disintegrate rapidly through buccal mucosa. It is desirable in the treatment of a number of diseases. Particularly, in epilepsy treatment in which it is very crucial to use drug on time and properly as described by the doctor. They are also advantageous for administrations of medicaments to patients who are traveling or have little access to water are similarly affected or patients who are mentally retarded, uncooperative, nauseated, or patients who have difficulties swallowing other dosage forms.

It is difficult to develop orally disintegrating compositions because of several different reasons and requirements such as disintegration, stability and taste masking properties. Dosage form must disintegrate in the oral cavity with the existence of saliva in a short period of time. So those compositions should have a porous structure. However, these porous characteristic tend to be very sensitive to humidity and may be lead to stability problems.

Moreover, effective taste masking technologies should also be adopted for bitter taste of active ingredients.

To fulfill all these requirements, the formulation for lacosamide needs to be adapted in particular by a careful selection of the excipients which give a high porous structure with high stability and suitable disintegration time. Additionally, to overcome bitter taste problem, taste masking have to be taken into consideration during the process.

### Description of the invention

The present invention provides a pharmaceutical formulation comprising a therapeutically effective amount of lacosamide or a pharmaceutically acceptable salt thereof in the form of orally disintegrating tablet.

The main object of the present invention is to obtain rapidly disintegrating, stable and taste masked formulation that comprise lacosamide.

According to another embodiment, the formulation of lacosamide is disintegrated in less than 1 min.

According to another object of the present invention is to provide the orally disintegrating composition wherein lacosamide or a pharmaceutically acceptable salt thereof is present in an amount of about 5 to 80 % by weight of total composition, preferably it is about 20 to 60 % by weight of total composition.

The main embodiment of the present invention is to provide an orally disintegrating composition comprising lacosamide or a pharmaceutically acceptable salt thereof and one or more pharmaceutically acceptable excipients.

The orally disintegrating compositions of this invention comprising at least one pharmaceutically acceptable excipient selected from the group comprising super disintegrants, taste masking coating, diluents, binders, lubricants, glidants, sweeteners and flavouring agents.

Another object of the present invention is to provide a formulation comprising crospovidone as a super-disintegrant which avoids the humidity, stability and bitter taste problems.

As used herein, the term "super-disintegrant" is defined as the pharmaceutical ingredient that provides improved compressibility and compatibility and achieves disintegration substantially faster than the conventional disintegrants. According to this embodiment, the term "super-disintegrant" is selected from the group comprising crospovidone, croscarmellose sodium, sodium carboxymethyl starch, sodium starch glycolate, soy polysaccharide, cross-linked alginic acid, crospovidone, gellan gum, xanthan gum, calcium silicate or ion exchange resins, preferably crospovidone.

It has been suprisingly found that in this orally disintegrating composition having a weight ratio of lacosamide to super disintegrants, particularly crospovidone, in the range of between 1 and 15 (w/w), preferably 2 and 6 (w/w).

The amount of crospovidone is present in an amount of 5 to 25 % by weight of total composition, preferably it is 8 to 15% by weight of total composition.

In one embodiment, granules are coated during the process, to overcome bitter taste of lacosamide. The taste-masking coating comprising aminoalkyl metacrylate, carboxymethylcellulose calcium, carboxymethylcellulose sodium, carnauba wax, cellulose acetate, cellulose acetate phthalate, ceresin, cetyl alcohol, chitosan, ethylcellulose, fructose, gelatin, glycerin, glyceryl behenate, glyceryl palmitostearate, hydroxyethyl cellulose, hydroxyethylmethyl cellulose, hydroxypropyl cellulose, hypromellose, hypromellose phthalate, isomalt, liquid glucose, maltitol, maltodextrin, methylcellulose, microcrystalline wax, paraffin, poloxamer, polydextrose, polyethylene glycol, polyethylene oxide, poly-DL-(lactic acid), polyvinyl acetate phthalate, polyvinyl alcohol, potassium chloride, povidone, shellac, shellac with stearic acid, sucrose, surface color agents, titanium oxide, tributyl citrate, triethyl citrate, vanillin, white wax, xylitol, yellow wax, zein or their copolymers or their mixtures.

The taste-masking coating preferably comprises dimethylaminoethyl methacrylate, butyl methacrylate and methyl methacrylate (Eudragit E 100) (Poly(butyl methacrylate-co-(2-demethylaminoeethyl)methacrylate-co-methyl methacrylate)) or mixture of polyethylene glycol and polyvinyl alcohol (Kollicoat IR).

The taste-masking coating is in the range of 1.0 to 50.0% by weight of total composition, preferably it is 2 to 30.0% by weight of total composition.

The diluents are selected from the group comprising microcrystalline cellulose, mannitol, spray-dried mannitol, lactose, starch, sodium carbonate, sodium bicarbonate, calcium carbonate and the like and mixtures thereof; preferably the diluents is mannitol.

The amount of mannitol is in the range of 1.0 to 60.0% by weight of total composition, preferably it is 10.0 to 40.0% by weight of total composition.

The binders are selected from the group comprising carbomers, carboxymethylcellulose sodium, cellulose acetate phthalate, chitosan, copovidone, corn starch and pregelatinized starch, dextrates, dextrin, dextrose, ethylcellulose, gelatin, glyceryl behenate, guar gum, hydrogenated vegetable oil type I, hydroxyethyl cellulose, hydroxyethylmethyl cellulose, hydroxypropyl cellulose, hydroxypropyl starch, hypromellose, liquid glucose, magnesium aluminum silicate, maltodextrin, maltose, methylcellulose, pectin, poloxamer, polycarbophil, polydextrose, polyethylene oxide, polymethacrylates, povidone, sodium alginate, starch, pregelatinized starch, stearic acid, sucrose or mixtures thereof.

The lubricants are selected from the group comprising magnesium strearate, calcium stearate, sodium stearyl fumarate, sodium lauryl sulphate, stearic acid and the like and mixtures thereof; preferably sodium stearyl fumarate.

The glidants selected from the group comprising colloidal silicon dioxide, colloidal anhydrous silica, talc, aluminium silicate and the like or mixtures thereof; preferably colloidal anhydrous silica.

The sweetners are selected from the group comprising aspartame, sucralose, saccharin, sugars such as glucose, lactose, fructose and sugar alcohols such as mannitol, sorbitol, xylitol, erythritol and the like or mixtures thereof; preferably sucralose.

The flavouring agents are selected from the group comprising menthol, peppermint, cinnamon, chocolate, vanillin and fruit essences such as cherry, orange, strawberry, grape etc. and the like or mixtures thereof; preferably strawberry.

In this present invention, to improve tablet compressibility and achieve faster disintegration, this orally disintegrating tablet composition has been designed, made up of the following:
- 5 to 80% by weight of lacosamide or pharmaceutically acceptable salt thereof,
- 1 to 60 % by weight of mannitol powder,
- 5 to 25 % by weight of crospovidone,
- 0.5 to 5 % by weight of flavor,
- 0.1 to 3 % by weight of sucralose,
- 0.01 to 5 % by weight of colloidal anhydrous silica,
- 0.1 to 5 % by weight of sodium stearyl fumarate,
- Preferably, taste masking coating,
- 5 to 80% by weight of lacosamide or pharmaceutically acceptable salt thereof,
- 1 to 60 % by weight of mannitol powder,
- 5 to 25 % by weight of crospovidone,
- 1 to 5 % by weight of povidone,
- 0.5 to 5 % by weight of flavor,
- 0.1 to 3 % by weight of sucralose,
- 0.01 to 5 % by weight of colloidal anhydrous silica,
- 0.1 to 5 % by weight of sodium stearyl fumarate,
- Preferably, taste masking coating
- 5 to 80% by weight of lacosamide or pharmaceutically acceptable salt thereof,
- 10 to 40 % by weight of granulated mannitol,
- 5 to 25 % by weight of crospovidone,
- 0.5 to 5 % by weight of flavor,
- 0.1 to 3 % by weight of sucralose,
- 0.1 to 5 % by weight of sodium stearyl fumarate,
- 1 to 50 % taste masking coating.
- 20 to 60% by weight of lacosamide or pharmaceutically acceptable salt thereof,
- 10 to 40 % by weight of mannitol powder,
- 8 to 15 % by weight of crospovidone,
- 0.5 to 5 % by weight of flavor,
- 0.1 to 3 % by weight of sucralose,
- 0.01 to 5 % by weight of colloidal anhydrous silica,
- 0.1 to 5 % by weight of sodium stearyl fumarate,
- Preferably, taste masking coating,
- 20 to 60% by weight of lacosamide or pharmaceutically acceptable salt thereof,
- 10 to 40 % by weight of mannitol powder,
- 8 to 15 % by weight of crospovidone,
- 1 to 5 % by weight of povidone,
- 0.5 to 5 % by weight of flavor,
- 0.1 to 3 % by weight of sucralose,
- 0.01 to 5 % by weight of colloidal anhydrous silica,
- 0.1 to 5 % by weight of sodium stearyl fumarate,
- Preferably, taste masking coating
and
- 20 to 60% by weight of lacosamide or pharmaceutically acceptable salt thereof,
- 10 to 40 % by weight of granulated mannitol,
- 8 to 15 % by weight of crospovidone,
- 0.5 to 5 % by weight of flavor,
- 0.1 to 3 % by weight of sucralose,
- 0.1 to 5 % by weight of sodium stearyl fumarate,
- 1 to 50 % taste masking coating.

The invention is further defined by reference of the following examples. Although the examples are not intended to limit the scope of the present invention, it should be considered in the light of the description detailed above.

**Example 1: Orally Disintegrating Tablet (wet granulation)**

| **ingredients** | **amount (%)** |
|---|---|
| lacosamide | 50.00 |
| mannitol powder | 33.16 |
| crospovidone | 14.00 |
| flavor | 1.00 |
| sucralose | 0.24 |
| colloidal anhydrous silica | 0.10 |
| sodyum stearil fumarat | 1.50 |

The production of the formulation is carried out as follows: lacosamide, mannitol powder and 50% of crospovidone are taken into granulator and mixed for 5 min. Granulation is achieved in suitable amount of water and granules are dried in fluid bed dryer or oven. Dried granules are sieved and strawberry flavor, sucralose, colloidal anhydrous silica and 50% of crospovidone are added. Mixed for 5 min. Sodium stearyl fumarate was added and mixed for 3 min. and pressed.

**Example 2: Orally Disintegrating Tablet (wet granulation)**

| **ingredients** | **amount (%)** |
|---|---|
| lacosamide | 40.00 |
| mannitol powder | 43.16 |
| crospovidone | 14.00 |
| flavor | 1.00 |
| sucralose | 0.24 |
| colloidal anhydrous silica | 0.10 |
| sodyum stearil fumarat | 1.50 |

The production of the formulation is carried out as follows: lacosamide, mannitol powder and 50% of crospovidone are taken into granulator and mixed for 5 min. Granulation is achieved in suitable amount of water and granules are dried in fluid bed dryer or oven. Dried granules are sieved and strawberry flavor, sucralose, colloidal anhydrous silica and 50% of crospovidone are added. Mixed for 5 min. Sodium stearyl fumarate was added and mixed for 3 min. and pressed.

**Example 3: Orally Disintegrating Tablet (dry granulation)**

| **ingredients** | **amount (%)** |
|---|---|
| lacosamide | 50.00 |
| mannitol powder | 30.16 |
| crospovidone | 14.00 |
| Povidone K25 | 3.00 |
| flavor | 1.00 |
| sucralose | 0.24 |
| colloidal anhydrous silica | 0.10 |
| sodyum stearil fumarat | 1.50 |

The production of the formulation is carried out as follows: lacosamide, mannitol powder, povidone K25 and 50% of crospovidone passed through the compactor or pressed in slug briquettes. Then sieved. Strawberry flavor, sucralose, colloidal anhydrous silica and 50% of crospovidone are added and mixed for 5 min. Sodium stearyl fumarate was added and mixed for 3 min. and pressed.

**Example 4: Orally Disintegrating Tablet (wet granulation)**

| **ingredients** | **amount (%)** |
|---|---|
| lacosamide | 50.00 |
| mannitol powder | 30.16 |
| crospovidone | 14.00 |
| Povidone K25 | 3.00 |
| flavor | 1.00 |
| sucralose | 0.24 |
| colloidal anhydrous silica | 0.10 |
| sodyum stearil fumarat | 1.50 |

The production of the formulation is carried out as follows: lacosamide, mannitol powder and 50% of crospovidone are taken into granulator and mixed for 5 min. Granulation is achieved in povidone solution prepared with suitable amount of water. Granules are dried in fluid bed dryer or oven. Dried granules are sieved and strawberry flavor, sucralose, colloidal anhydrous silica and 50% of crospovidone are added. Mixed for 5 min. Sodium stearyl fumarate was added and mixed for 3 min. and pressed.

**Example 5: Orally Disintegrating Tablet (wet granulation)**

| **ingredients** | **amount (%)** |
|---|---|
| lacosamide | 60.00 |
| mannitol powder | 20.16 |
| crospovidone | 14.00 |
| Povidone K25 | 3.00 |
| flavor | 1.00 |
| sucralose | 0.24 |
| colloidal anhydrous silica | 0.10 |
| sodyum stearil fumarat | 1.50 |

The production of the formulation is carried out as follows: lacosamide, mannitol powder and 50% of crospovidone are taken into granulator and mixed for 5 min. Granulation is achieved in povidone K25 solution prepared with suitable amount of water. Granules are dried in fluid bed dryer or oven. Dried granules are sieved and strawberry flavor, sucralose, colloidal anhydrous silica and 50% of crospovidone are added. Mixed for 5 min. Sodium stearyl fumarate was added and mixed for 3 min. and pressed.

**Example 6: Orally Disintegrating Tablet (granulation and granule coating)**

| **ingredients** | **amount (%)** |
|---|---|
| lacosamide | 50.00 |
| mannitol powder | 28.16 |
| crospovidone | 14.00 |
| Povidone K25 | 2.00 |
| Eudragit E 100 | 3.00 |
| flavor | 1.00 |
| sucralose | 0.24 |
| colloidal anhydrous silica | 0.10 |
| sodyum stearil fumarat | 1.50 |

The production of the formulation is carried out as follows: lacosamide, mannitol powder and 50% of crospovidone are taken into granulator and mixed for 5 min. Granulation is achieved in povidone K25 solution prepared with suitable amount of water. Granules are dried in fluid bed dryer or oven. Dried granules are sieved and sieved granules are coated in fluid bed dryer with Eudragit E 100 solution prepared with suitable amount of water. Strawberry flavor, sucralose, colloidal anhydrous silica and 50% of crospovidone are added. Mixed for 5 min. Sodium stearyl fumarate was added and mixed for 3 min. and pressed.

**Example 7: Orally Disintegrating Tablet (powder granule coating)**

| **ingredients** | **amount (%)** |
|---|---|
| lacosamide | 50.00 |
| mannitol powder | 30.16 |
| crospovidone | 14.00 |
| Kollicoat IR | 3.00 |
| flavor | 1.00 |
| sucralose | 0.24 |
| colloidal anhydrous silica | 0.10 |
| sodyum stearil fumarat | 1.50 |

The production of the formulation is carried out as follows: lacosamide, mannitol powder and 50% of crospovidone are taken into fluid bed dryer. Powder mixture is coated - granulated with Kollicoat IR solution prepared with suitable amount of water and dried. Dried granules was sieved. Strawberry flavor, sucralose, colloidal anhydrous silica and 50% of crospovidone are added. Mixed for 5 min. Sodium stearyl fumarate was added and mixed for 3 min. and pressed.

**Example 8: Orally Disintegrating Tablet (hot - melt extrusion)**

| **ingredients** | **amount (%)** |
|---|---|
| lacosamide | 33.33 |
| granulated mannitol | 22.17 |
| crospovidone | 9.34 |
| Eudragit E 100 | 33.33 |
| flavor | 0.67 |
| sucralose | 0.16 |
| sodyum stearil fumarat | 1.00 |

The production of the formulation is carried out as follows: lacosamide and Eudragit E 100 are taken into an extruder. Extrudes are obtained via hot-melt extrusion technique. Extrudes are cut and pellets are obtained. Mannitol powder, crospovidone, flavor, sucralose are added to pellets and mixed for 5 min. Sodium stearyl fumarate was added to mixed granules and mixed for 3 min. and pressed.

## Claims

1. An orally disintegrating composition comprising lacosamide or a pharmaceutically acceptable salt thereof, a superdisintegrant and one or more pharmaceutically acceptable excipients.

2. The orally disintegrating composition according to claim 1, wherein the formulation of lacosamide is disintegrated in less than 1 min.

3. The orally disintegrating composition according to claim 1, wherein lacosamide or a pharmaceutically acceptable salt thereof is present in an amount of about 5 to 80 % by weight of total composition, preferably it is about 20 to 60 % by weight of total composition.

4. The orally disintegrating composition according to claims 1, wherein the one or more pharmaceutically acceptable excipients are selected from the group comprising taste masking coating, diluents, binders, lubricants, glidants, sweeteners and flavouring agents.

5. The orally disintegrating composition according to claim 1, wherein the super disintegrants are selected from the group comprising crospovidone, croscarmellose sodium, sodium carboxymethyl starch, sodium starch glycolate, soy polysaccharide, cross-linked alginic acid, crospovidone, gellan gum, xanthan gum, calcium silicate or ion exchange resins, low-substituted hydroxypropylcellulose, sodium starch glycollate and mixtures thereof.

6. The orally disintegrating composition according to claim 1, wherein the weight ratio of lacosamide to super disintegrants is in the range of between 1 and 15 (w/w), preferably 2 and 6 (w/w).

7. The orally disintegrating composition according to claim 5 and 6, wherein the super disintegrant is preferably crospovidone.

8. The orally disintegrating composition according to claim 7, wherein the amount of crospovidone is present in an amount of 5 to 25 % by weight of total composition, preferably it is 8 to 15% by weight of total composition.

9. The orally disintegrating composition according to claim 4, wherein taste-masking coating comprising aminoalkyl metacrylate, carboxymethylcellulose calcium, carboxymethylcellulose sodium, carnauba wax, cellulose acetate, cellulose acetate phthalate, ceresin, cetyl alcohol, chitosan, ethylcellulose, fructose, gelatin, glycerin, glyceryl behenate, glyceryl palmitostearate, hydroxyethyl cellulose, hydroxyethylmethyl cellulose, hydroxypropyl cellulose, hypromellose, hypromellose phthalate, isomalt, liquid glucose, maltitol, maltodextrin, methylcellulose, microcrystalline wax, paraffin, poloxamer, polydextrose, polyethylene glycol, polyethylene oxide, poly-DL-(lactic acid), polyvinyl acetate phthalate, polyvinyl alcohol, potassium chloride, povidone, shellac, shellac with stearic acid, sucrose, surface color agents, titanium oxide, tributyl citrate, triethyl citrate, dimethylaminoethyl methacrylate, butyl methacrylate, methyl methacrylate, vanillin, white wax, xylitol, yellow wax, zein and their copolymers or their mixtures thereof.

10. The orally disintegrating composition according to claim 9, wherein the taste-masking coating is copolymer of dimethylaminoethyl methacrylate, butyl methacrylate and methyl methacrylate or mixture of polyethylene glycol and polyvinyl alcohol.

11. The orally disintegrating composition according to claim 9 and 10, wherein the amount of taste-masking coating is in the range of 1.0 to 50.0% by weight of total composition, preferably it is 2 to 30.0% by weight of total composition.

12. The orally disintegrating composition according to claim 4, wherein the diluents are selected from the group comprising microcrystalline cellulose, mannitol, spray-dried mannitol, lactose, starch, sodium carbonate, sodium bicarbonate, calcium carbonate or mixtures thereof; preferably the diluents is mannitol.

13. The orally disintegrating composition according to claim 12, wherein the amount of mannitol is in an amount of 1.0 to 60.0% by weight of total composition, preferably it is 10.0 to 40.0% by weight of total composition.

14. The orally disintegrating composition according to claim 4, wherein the binders are selected from the group comprising carbomers, carboxymethylcellulose sodium, cellulose acetate phthalate, chitosan, copovidone, corn starch and pregelatinized starch, dextrates, dextrin, dextrose, ethylcellulose, gelatin, glyceryl behenate, guar gum, hydrogenated vegetable oil type I, hydroxyethyl cellulose, hydroxyethylmethyl cellulose, hydroxypropyl cellulose, hydroxypropyl starch, hypromellose, liquid glucose, magnesium aluminum silicate, maltodextrin, maltose, methylcellulose, pectin, poloxamer, polycarbophil, polydextrose, polyethylene oxide, polymethacrylates, povidone, sodium alginate, starch, pregelatinized starch, stearic acid, sucrose or mixtures thereof.

15. The orally disintegrating composition according to claim 4, wherein the lubricants are selected from the group comprising magnesium strearate, calcium stearate, sodium stearyl fumarate, sodium lauryl sulphate, stearic acid and mixtures thereof.

16. The orally disintegrating composition according to claim 4, wherein the glidants selected from the group comprising colloidal silicon dioxide, colloidal anhydrous silica, talc, aluminium silicate and mixtures thereof; preferably colloidal anhydrous silica.

17. The orally disintegrating composition according to claim 4, wherein the sweetners are selected from the group comprising aspartame, sucralose, saccharin, sugars such as glucose, lactose, fructose and sugar alcohols such as mannitol, sorbitol, xylitol, erythritol and mixtures thereof; preferably sucralose.

18. The orally disintegrating composition according to claim 4, wherein the flavouring agents are selected from the group comprising menthol, peppermint, cinnamon, chocolate, vanillin and fruit essences such as cherry, orange, strawberry, grape etc. and mixtures thereof; preferably strawberry.

19. The orally disintegrating composition according to any preceding claim comprising;
- 5 to 80% by weight of lacosamide or pharmaceutically acceptable salt thereof,
- 1 to 60 % by weight of mannitol powder,
- 5 to 25 % by weight of crospovidone,
- 0.5 to 5 % by weight of flavor,
- 0.1 to 3 % by weight of sucralose,
- 0.01 to 5 % by weight of colloidal anhydrous silica,
- 0.1 to 5 % by weight of sodium stearyl fumarate,
- Preferably, taste masking coating.

20. The orally disintegrating composition according to any preceding claim comprising;
- 5 to 80% by weight of lacosamide or pharmaceutically acceptable salt thereof,
- 1 to 60 % by weight of mannitol powder,
- 5 to 25 % by weight of crospovidone,
- 1 to 5 % by weight of povidone,
- 0.5 to 5 % by weight of flavor,
- 0.1 to 3 % by weight of sucralose,
- 0.01 to 5 % by weight of colloidal anhydrous silica,
- 0.1 to 5 % by weight of sodium stearyl fumarate,
- Preferably, taste masking coating.

21. The orally disintegrating composition according to any preceding claim comprising;
- 5 to 80% by weight of lacosamide or pharmaceutically acceptable salt thereof,
- 1 to 60 % by weight of granulated mannitol,
- 5 to 25 % by weight of crospovidone,
- 0.5 to 5 % by weight of flavor,
- 0.1 to 3 % by weight of sucralose,
- 0.1 to 5 % by weight of sodium stearyl fumarate,
- 1 to 50 % taste masking coating.

## Patentansprüche

1. Orale, zerfallende Zusammensetzung, umfassend Lacosamid oder ein pharmazeutisch annehmbares Salz davon, ein Super-Zerfallmittel und ein oder mehrere pharmazeutisch annehmbare Hilfsstoffe.

2. Orale, zerfallende Zusammensetzung gemäß Anspruch 1, worin die Formulierung mit Lacosamid in weniger als 1 min zerfällt.

3. Orale, zerfallende Zusammensetzung gemäß Anspruch 1, worin Lacosamid oder ein pharmazeutisch annehmbares Salz davon in einer Menge von etwa 5 bis 80 Gew.-% der Gesamtzusammensetzung vorhanden ist, vorzugsweise von etwa 20 bis 60 Gew.-% der Gesamtzusammensetzung.

4. Orale, zerfallende Zusammensetzung gemäß Anspruch 1, worin der eine oder die mehreren pharmazeutisch annehmbaren Hilfsstoffe ausgewählt sind aus der Gruppe bestehend aus geschmacksmaskierender Beschichtung, Verdünnungsmitteln, Bindermitteln, Schmiermitteln, Gleitmitteln, Süßstoffen und Geschmacksstoffen.

5. Orale, zerfallende Zusammensetzung gemäß Anspruch 1, worin das Super-Zerfallmittel ausgewählt ist aus der Gruppe bestehend aus Crospovidon, Croscarmellosenatrium, Natriumcarboxymethylstärke, Natriumstärkeglykolat, Sojapolysaccharid, vernetzte Alginsäure, Crospovidon, Gellangummi, Xanthangummi, Kalziumsilikat oder Ionen-Austauschharze, niedrig substituierter Hydroxypropylzellulose, Natriumstärkeglykolat und Mischungen davon.

6. Orale, zerfallende Zusammensetzung gemäß Anspruch 1, worin das Gewichtsverhältnis Lacosamid zu Super-Zerfallmitteln in dem Bereich zwischen 1 und 15 (w/w), vorzugsweise 2 und 6 (w/w) ist.

7. Orale, zerfallende Zusammensetzung gemäß Anspruch 5 und 6, worin das Super-Zerfallmittel vorzugsweise Crospovidon ist.

8. Orale, zerfallende Zusammensetzung gemäß Anspruch 7, worin die Menge an Crospovidon in einer Menge von 5 bis 25 Gew.-% der Gesamtzusammensetzung, vorzugsweise 8 bis 15 Gew.-% der Gesamtzusammensetzung vorliegt.

9. Orale, zerfallende Zusammensetzung gemäß Anspruch 4, worin die geschmacksmaskierende Beschichtung Aminoalkylmethacrylat, Carboxymethylzellulose-Kalzium, Carboxymethylzellulose-Natrium, Carnauba-Wachs, Zelluloseacetat, Zelluloseacetatphthalat, Ceresin, Cetylalkohol, Chitosan, Ethylzellulose, Fructose, Gelatine, Glycerin, Glycerinbehenat, Glycerinpalmitostearat, Hydroxyethylzellulose, Hydroxyethylmethylzellulose, Hydroxypropylzellulose, Hypromellose, Hypromellosephthalat, Isomalt, flüssige Glucose, Maltitol, Maltodextrin, Methylzellulose, mikrokristallines Wachs, Paraffin, Poloxamer, Polydextrose, Polyethylenglykol, Polyethylenoxid, poly-DL-(Milchsäure), Polyvinylacetatphthalat, Polyvinylalkohol, Kaliumchlorid, Povidon, Schellack, Schellack mit Stearinsäure, Sucrose, Oberflächenfarbmittel, Titanoxid, Tributylcitrat, Triethylcitrat, Dimethylaminoethylmethacrylat, Butylmethacrylat, Methylmethacrylat, Vanillin, weißes Wachs, Xylitol, Gelbes Wachs, Zein und deren Copolymere oder deren Mischungen davon umfasst.

10. Orale, zerfallende Zusammensetzung gemäß Anspruch 9, worin die geschmacksmaskierende Beschichtung ein Copolymer aus Dimethylaminoethylmethacrylat, Butylmethacrylat und Methylmethacrylat oder Mischungen aus Polyethylenglykol und Polyvinylalkohol ist.

11. Orale, zerfallende Zusammensetzung gemäß Anspruch 9 und 10, worin die Menge der geschmacksmaskierenden Beschichtung in dem Bereich von 1,0 bis 50,0 Gew.-% der Gesamtzusammensetzung, vorzugsweise 2 bis 30,0 Gew.-% der Gesamtzusammensetzung ist.

12. Orale, zerfallende Zusammensetzung gemäß Anspruch 4, worin die Verdünnungsmittel ausgewählt sind aus der Gruppe bestehend aus mikrokristalliner Zellulose, Mannitol, sprühgetrocknetem Mannitol, Lactose, Stärke, Natriumcarbonat, Natriumbicarbonat, Kalziumkarbonat oder Mischungen davon; vorzugsweise ist das Verdünnungsmittel Mannitol.

13. Orale, zerfallende Zusammensetzung gemäß Anspruch 12, worin die Menge an Mannitol eine Menge von 1,0 bis 60,0 Gew.-% der Gesamtzusammensetzung, vorzugsweise 10,0 bis 40,0 Gew.-% der Gesamtzusammensetzung ist.

14. Orale, zerfallende Zusammensetzung gemäß Anspruch 4, worin die Bindemittel ausgewählt sind aus der Gruppe bestehend aus Carbomeren, Carboxymethylzellulose-Natrium, Zelluloseacetatphthalat, Chitosan, Copovidon, Maisstärke und vorgelatinierte Stärke, Dextrate, Dextrin, Dextrose, Ethylzellulose, Gelatine, Glycerinbehenat, Guargummi, hydrogeniertes Pflanzenöl Typ I, Hydroxyethylzellulose, Hydroxyethylmethylzellulose, Hydroxypropylzellulose, Hydroxypropylstärke, Hypromellose, flüssige Glucose, Magnesiumaluminiumsilikat, Maltodextrin, Maltose, Methylzellulose, Pektin, Poloxamer, Polycarbophil, Polydextrose, Polyethylenoxid, Polymethacrylaten, Povidon, Natriumalginat, Stärke, vorgelatinierter Stärke, Stearinsäure, Sucrose oder Mischungen davon.

15. Orale, zerfallende Zusammensetzung gemäß Anspruch 4, worin die Schmiermittel ausgewählt sind aus der Gruppe bestehend aus Magnesiumstearat, Kalziumstearat, Natriumstearylfumarat, Natriumlaurylsulfat, Stearinsäure und Mischungen davon.

16. Orale, zerfallende Zusammensetzung gemäß Anspruch 4, worin die Gleitmittel ausgewählt sind aus der Gruppe bestehend aus kolloidalem Siliziumdioxid, kolloidalem wasserfreiem Silica, Talg, Aluminiumsilikat und Mischungen davon; vorzugsweise kolloidales wasserfreies Silica.

17. Orale, zerfallende Zusammensetzung gemäß Anspruch 4, worin die Süßungsmittel ausgewählt sind aus der Gruppe bestehend aus Aspartam, Sucralose, Saccharin, Zucker wie Glucose, Lactose, Fructose und Zuckeralkohole wie Mannitol, Sorbitol, Xylitol, Erythritol und Mischungen davon; vorzugsweise Sucralose.

18. Orale, zerfallende Zusammensetzung gemäß Anspruch 4, worin die Geschmacksmittel ausgewählt sind aus der Gruppe bestehend aus Menthol, Pfefferminz, Zimt, Schokolade, Vanillin und Fruchtessenzen wie Kirsche, Orange, Erdbeere, Traube usw., und Mischungen davon; vorzugsweise Erdbeere.

19. Orale, zerfallende Zusammensetzung gemäß irgendeinem der vorhergehenden Ansprüche, umfassend:
- 5 bis 80 Gew.-% Lacosamid oder pharmazeutisch annehmbarer Salz davon,
- 1 bis 60 Gew.-% Mannitol-Pulver,
- 5 bis 25 Gew.-% Crospovidon,
- 0,5 bis 5 Gew.-% Geschmacksstoff,
- 0,1 bis 3 Gew.-% Sucralose,
- 0,01 bis 5 Gew.-% kolloidales wasserfreies Silica,
- 0,1 bis 5 Gew.-% Natriumstearylfumarat,
- vorzugsweise, geschmacksmaskierende Beschichtung.

20. Orale, zerfallende Zusammensetzung gemäß irgendeinem der vorhergehenden Ansprüche, umfassend:
- 5 bis 80 Gew.-% Lacosamid oder pharmazeutisch annehmbarer Salz davon,
- 1 bis 60 Gew.-% Mannitol-Pulver,
- 5 bis 25 Gew.-% Crospovidon,
- 1 bis 5 Gew.-% Povidon,
- 0,5 bis 5 Gew.-% Geschmackstoff,
- 0,1 bis 3 Gew.-% Sucralose,
- 0,01 bis 5 Gew.-% kolloidales wasserfreies Silica,
- 0,1 bis 5 Gew.-% Natriumstearylfumarat,
- vorzugsweise, geschmacksmaskierende Beschichtung.

21. Orale, zerfallende Zusammensetzung gemäß irgendeinem der vorhergehenden Ansprüche, umfassend:
- 5 bis 80 Gew.-% Lacosamid oder pharmazeutisch annehmbarer Salz davon,
- 1 bis 60 Gew.-% granuliertes Mannitol,
- 5 bis 25 Gew.-% Crospovidon,
- 0,5 bis 5 Gew.-% Geschmacksstoff,
- 0,1 bis 3 Gew.-% Sucralose,
- 0,1 bis 5 Gew.-% Natriumstearylfumarat,
- 1 bis 50 Gew.-% geschmacksmaskierende Beschichtung.

## Revendications

1. Composition à délitement oral comprenant du lacosamide ou un sel pharmaceutiquement acceptable de celui-ci, un superdésintégrant et un ou plusieurs excipients pharmaceutiquement acceptables.

2. Composition à délitement oral selon la revendication 1, dans laquelle la formulation de lacosamide se délite en moins de 1 minute.

3. Composition à délitement oral selon la revendication 1, dans laquelle le lacosamide ou un sel pharmaceutiquement acceptable de celui-ci est présent en une quantité d'environ 5 à 80 % en poids de la composition totale, de préférence elle est d'environ 20 à 60 % en poids de la composition totale.

4. Composition à délitement oral selon la revendication 1, dans laquelle l'un ou les plusieurs excipients pharmaceutiquement acceptable sont choisis dans le groupe comprenant un revêtement de masquage de goût, les diluants, les liants, les lubrifiants, les agents glissants, les édulcorants et les agents aromatisants.

5. Composition à délitement oral selon la revendication 1, dans laquelle les superdésintégrants sont choisis dans le groupe comprenant la crospovidone, la croscarmellose sodique, l'amidon carboxyméthylé sodique, le glycolate d'amidon sodique, un polysaccharide de soja, l'acide alginique réticulé, la crospovidone, la gomme gellane, la gomme xanthane, le silicate de calcium ou les résines échangeuses d'ions, l'hydroxypropylcellulose faiblement substituée, le glycolate d'amidon sodique et leurs mélanges.

6. Composition à délitement oral selon la revendication 1, dans laquelle le rapport pondéral lacosamide sur superdésintégrants est situé dans la plage entre 1 et 15 (en poids/poids), de préférence 2 et 6 (en poids/poids).

7. Composition à délitement oral selon la revendication 5 et 6, dans laquelle le superdésintégrant est de préférence la crospovidone.

8. Composition à délitement oral selon la revendication 7, dans laquelle la quantité de crospovidone est présente en une quantité de 5 à 25 % en poids de la composition totale, de préférence elle est de 8 à 15 % en poids de la composition totale.

9. Composition à délitement oral selon la revendication 4, dans laquelle le revêtement de masquage de goût comprend un méthacrylate d'aminoalkyle, de la carboxyméthyl-cellulose calcique, de la carboxyméthylcellulose sodique, de la cire de carnauba, de l'acétate de cellulose, du phtalate d'acétate de cellulose, de la cérésine, de l'alcool cétylique, du chitosan, de l'éthylcellulose, du fructose, de la gélatine, de la glycérine, du béhénate de glycéryle, du palmitostéarate de glycéryle, de l'hydroxyéthylcellulose, de l'hydroxyéthylméthylcellulose, de l'hydroxypropylcellulose, de l'hypromellose, du phtalate d'hypromellose, de l'isomalt, du glucose liquide, du maltitol, de la maltodextrine, de la méthylcellulose, de la cire microcristalline, de la paraffine, un poloxamère, un polydextrose, un polyéthylène-glycol, un polyoxyde d'éthylène, un poly-DL-(acide lactique), un phtalate d'acétate de polyvinyle, un polyalcool de vinyle, du chlorure de potassium, de la povidone, de la gomme laque, de la gomme laque avec de l'acide stéarique, du saccharose, des agents de couleur de surface, de l'oxyde de titane, du citrate de tributyle, du citrate de triéthyle, du méthacrylate de diméthylaminoéthyle, du méthacrylate de butyle, du méthacrylate de méthyle, de la vanilline, de la cire blanche, du xylitol, de la cire jaune, de la zéine et leurs copolymères ou leurs mélanges.

10. Composition à délitement oral selon la revendication 9, dans laquelle le revêtement de masquage de goût est un copolymère de méthacrylate de diméthylaminoéthyle, de méthacrylate de butyle et de méthacrylate de méthyle ou un mélange de polyéthylène-glycol et de polyalcool de vinyle.

11. Composition à délitement oral selon la revendication 9 et 10, dans laquelle la quantité de revêtement de masquage de goût est située dans la plage allant de 1,0 à 50,0 % en poids de la composition totale, de préférence elle est de 2 à 30,0 % en poids de la composition totale.

12. Composition à délitement oral selon la revendication 4, dans laquelle les diluants sont choisis dans le groupe comprenant la cellulose microcristalline, le mannitol, le mannitol séché par atomisation, le lactose, l'amidon, le carbonate de sodium, le bicarbonate de sodium, le carbonate de calcium ou leurs mélanges ; de préférence, les diluants sont le mannitol.

13. Composition à délitement oral selon la revendication 12, dans laquelle la quantité de mannitol est en une quantité de 1,0 à 60,0 % en poids de la composition totale, de préférence elle est de 10,0 à 40,0 % en poids de la composition totale.

14. Composition à délitement oral selon la revendication 4, dans laquelle les liants sont choisis dans le groupe comprenant les carbomères, la carboxyméthylcellulose sodique, le phtalate d'acétate de cellulose, le chitosan, la copovidone, l'amidon de maïs et l'amidon prégélatinisé, les dextrates, la dextrine, le dextrose, l'éthylcellulose, la gélatine, le béhénate de glycéryle, la gomme guar, une huile végétale hydrogénée de type I, l'hydroxyéthyl-cellulose, l'hydroxyéthylméthylcellulose, l'hydroxy-propylcellulose, l'amidon hydroxypropylé, l'hypromellose, le glucose liquide, le silicate de magnésium et d'aluminium, la maltodextrine, le maltose, la méthylcellulose, la pectine, un poloxamère, un polycarbophile, un polydextrose, un polyoxyde d'éthylène, les polyméthacrylates, la povidone, l'alginate de sodium, l'amidon, l'amidon prégélatinisé, l'acide stéarique, le saccharose ou leurs mélanges.

15. Composition à délitement oral selon la revendication 4, dans laquelle les lubrifiants sont choisis dans le groupe comprenant le stéarate de magnésium, le stéarate de calcium, le fumarate de stéaryle sodique, le lauryl-sulfate de sodium, l'acide stéarique et leurs mélanges.

16. Composition à délitement oral selon la revendication 4, dans laquelle les agents glissants sont choisis dans le groupe comprenant le dioxyde de silicium colloïdal, la silice colloïdale anhydre, le talc, le silicate d'aluminium et leurs mélanges ; de préférence la silice colloïdale anhydre.

17. Composition à délitement oral selon la revendication 4, dans laquelle les édulcorants sont choisis dans le groupe comprenant l'aspartame, le sucralose, la saccharine, les sucres tels que le glucose, le lactose, le fructose et les alcools de sucre tels que le mannitol, le sorbitol, le xylitol, l'érythritol et leurs mélanges ; de préférence le sucralose.

18. Composition à délitement oral selon la revendication 4, dans laquelle les agents aromatisants sont choisis dans le groupe comprenant le menthol, la menthe poivrée, la cannelle, le chocolat, la vanilline et les essences de fruits telles que la cerise, l'orange, la fraise, le raisin, etc., et leurs mélanges ; de préférence la fraise.

19. Composition à délitement oral selon l'une quelconque des revendications précédentes comprenant ;
- 5 à 80 % en poids de lacosamide ou d'un sel pharmaceutiquement acceptable de celui-ci,
- 1 à 60 % en poids de poudre de mannitol,
- 5 à 25 % en poids de crospovidone,
- 0,5 à 5 % en poids d'un arôme,
- 0,1 à 3 % en poids de sucralose,
- 0,01 à 5 % en poids de silice colloïdale anhydre,
- 0,1 à 5 % en poids de fumarate de stéaryle sodique,
- de préférence, un revêtement de masquage de goût.

20. Composition à délitement oral selon l'une quelconque des revendications précédentes comprenant ;
- 5 à 80 % en poids de lacosamide ou d'un sel pharmaceutiquement acceptable de celui-ci,
- 1 à 60 % en poids de poudre de mannitol,
- 5 à 25 % en poids de crospovidone,
- 1 à 5 % en poids de povidone,
- 0,5 à 5 % en poids d'un arôme,
- 0,1 à 3 % en poids de sucralose,
- 0,01 à 5 % en poids de silice colloïdale anhydre,
- 0,1 à 5 % en poids de fumarate de stéaryle sodique,
- de préférence, un revêtement de masquage de goût.

21. Composition à délitement oral selon l'une quelconque des revendications précédentes comprenant ;
- 5 à 80 % en poids de lacosamide ou d'un sel pharmaceutiquement acceptable de celui-ci,
- 1 à 60 % en poids de mannitol granulé,
- 5 à 25 % en poids de crospovidone,
- 0,5 à 5 % en poids d'un arôme,
- 0,1 à 3 % en poids de sucralose,
- 0,1 à 5 % en poids de fumarate de stéaryle sodique,
- 1 à 50 % d'un revêtement de masquage de goût.
